# EUROPEAN PATENT SPECIFICATION

(11) **EP 2 069 769 B1**
(45) Date of publication and mention of the grant of the patent: **10.02.2010**
(21) Application number: 07804343.7
(22) Date of filing: 19.09.2007
(51) Int. Cl.: G01N 24/08, G01N 33/543, G01R 33/465

(54) **METHOD OF DETECTING INTERACTIONS ON A MICROARRAY USING NUCLEAR MAGNETIC RESONANCE**
VERFAHREN ZUM NACHWEIS VON WECHSELWIRKUNGEN AUF EINEM MIKROARRAY MITTELS KERNMAGNETRESONANZ
PROCEDE DE DETECTION D'INTERACTIONS SUR UN JEU ORDONNE PAR RESONANCE MAGNETIQUE NUCLEAIRE (RMN)

(30) Priority: 20.09.2006 GB 0618514
(43) Date of publication of application: 17.06.2009
(73) Proprietor: The Nottingham Trent University, Nottingham, Nottinghamshire NG11 8NS (GB)
(72) Inventor: MCHALE, Glen, Nottinghamshire NG8 2BP (GB); NEWTON, Michael Ian, Nottinghamshire NG5 4EA (GB); BENCSIK, Martin, Nottinghamshire NG12 3NB (GB); CAVE, Gareth, Wynn, Vaughan, Conwy LL22 9AF (GB)
(74) Representative: Kiddle, Simon John
(86) International application number: PCT/GB2007/003562
(87) International publication number: WO 2008/035073

(56) References cited:
- WO-A-02/087632
- WO-A-2005/061724
- WO-A-2006/122083
- US-A1- 2003 092 029
- HÖGEMANN D ET AL: "High throughput magnetic resonance imaging for evaluating targeted nanoparticle probes" BIOCONJUGATE CHEMISTRY, ACS, WASHINGTON, DC, US, vol. 13, no. 1, 1 January 2002 (2002-01-01), pages 116-121, XP002281380 ISSN: 1043-1802
- MANUEL PEREZ J ET AL: "Use of magnetic nanoparticles as nanosensors to probe for molecular interactions" CHEMBIOCHEM - A EUROPEAN JOURNAL OF CHEMICAL BIOLOGY, WILEY VCH, WEINHEIM, DE, vol. 5, 26 February 2004 (2004-02-26), pages 261-264, XP002418115 ISSN: 1439-4227
- MANUEL PEREZ J ET AL: "Magnetic relaxation switches capable of sensing molecular interactions" NATURE BIOTECHNOLOGY, NATURE PUBLISHING GROUP, NEW YORK, NY, US, vol. 20, 1 August 2002 (2002-08-01), pages 816-820, XP002418116 ISSN: 1087-0156 cited in the application

## Description

### Field of the Invention

The present invention relates to methods of using nuclear magnetic resonance (NMR) to detect the interaction of target substances with probes present at locations of a microarray.

### Background of the Invention

Microarrays are libraries of biological or chemical entities immobilised in a grid/array on a solid surface and methods for making and using microarrays are well known in the art. A variation on this theme is immobilisation of these entities onto beads, which are then formed into a grid/array. The entities immobilised in the array can be referred to as probes. These probes interact with targets (a gene, mRNA, cDNA, protein, etc) and the extent of interaction is assessed using fluorescent labels, colorimetric/chromogenic labels, radioisotope labels or label-free methods (e.g. scanning Kelvin microscopy, mass spectrometry, surface plasmon resonance, etc). The interaction may include binding, hybridization, absorption or adsorption. The microarray process provides a combinatorial approach to assessing interactions between probes and targets. The basic microarray concept is described by the Oxford Gene Technologies US Patent No: 5700637 and 6054270.

One type of array uses nucleic acid molecules as the probes. A DNA microarray is a collection of microscopic DNA spots attached to a solid substrate, e.g. glass, plastic or silicon chip, forming an array. DNA microarrays are now commercially available. There are three basic forms: spotted microarrays, lithographic microarrays and a bead-based systems. Each involves analysing DNA sequences by the immobilisation of cDNA probes or *in situ* creation of oligonucleotide sequences and subsequent hybridisation with target mRNA/cDNA complementary to the probes. Often the target cDNA are fluorescently labelled. Sequencing by hybridization approaches are described, for example, in US Patent No: 6913879, 6025136, 6018041, 5525464 and 5202231.

Two approaches exist to the creation and immobilisation of DNA probes. In the first approach oligonucleotide sequences are built *in situ* base by base on the chip. In the second, cDNA or oligonucleotide probes are deposited on the array using contact or non-contact printing methods.

In the spotted microarray approach, oligonucleotides, cDNA or small fragments of PCR products corresponding to mRNAs are printed in an array pattern on a solid substrate by either a spotting robot using pins or variations on ink-jet printing methods. The spots are typically in the 30-500 µm size range with separations of the order of 100 µm or more. A lack of uniformity of spot size, variations of spot shape and donut or ring-stain patterns caused during the drying of spots can result in non-uniform immobilisation of the DNA and hence non-uniform fluorescence following the hybridisation.

In lithographic microarrays, sequences of oligonucleotides (A, C, T, G) are built up by selective protection and deprotection of localised areas of the substrate. This approach has been employed, inter alia, by Affymetrix. Affymetrix chips generally provide higher probe densities (spot sizes of the order of 10 µm or greater), but have shorter sequence lengths than in spotted or bead microarrays. The fluorescent labelling of target cDNA remains a key part of the detection strategy. The photolithographic approach is described in US Patent No: 6045996 and 5143854.

An alternative method for making arrays employs bead based microarrays. An example of this approach is the system used by Illumina (http://www.illumina.com/) in which probes are immobilised on small (3-5 µm diameter) beads. After hybridisation the beads are cast onto a surface and drawn into wells by surface tension. In the Illumina system, the wells are etched into the ends of optical fibers in fiber bundles. The fluorescence signal is then read for each bead. The method includes a tagging of each bead so that the bioactive agent on each bead can be decoded from the probe position and a decoding system system is needed to distinguish the different probes used. The bead based system is described in US Patent No: 6023540, 6327410, 6266459, 6620584 and 7033754.

Whilst the above descriptions relate to DNA microarrays, the same principles have been extended to protein and chemical microarrays. In these cases the probes immobilised on the surface are specific proteins, antibodies, small molecule compounds, peptides, carbohydrates, etc rather than DNA sequences. The targets are complex analytes, such as serum, total cell extracts, and.whole blood. The key concepts of an array of probes, which undergo selective binding/interaction with a target and which are then interrogated via, for example, a fluorescent signal remain central to the method.

A review of ideas on protein and chemical microarrays is given by Xu and Lam in "Protein and Chemical Microarrays-Powerful Tools for Proteomics", J Biomed., 2003(5): 257-266, 2003. This reference also provides the historical sequence in the development of DNA microarrays. Current research is also extending the microarray concept to include microarrays of cells. A review of patent issues related to early microarrays is given by Rouse and Hardiman ("Microarray technology - an intellectual property retrospective", Pharmacogenomics, 4(5): 623-632, 2003).

As will be apparent from the discussion provided above, different ways of interrogating arrays have been used in the prior art. The most common form of interrogation is to use fluorescent confocal microscopy to obtain an integrated intensity signal for each spot on the substrate or each bead, e.g. by labelling the sample applied to the array. Changes in signal intensity across the array provide information about hybridisation. Fluorescence based techniques are currently the most common methods used. However, radioisotope labelling and a range of label-free methods, e.g. scanning Kelvin microscopy, scanning tunnelling microscopy, electrochemical detection, are also possible. By way of example, US Patent Application No: 2006/089825, 2004/0029131 and 2003/0175945 describe a scanning Kelvin microprobe (SKM) approach to biomolecular interactions and assays with biomolecules immobilised on a substrate. US Patent Application No: 2005/0258821 and 2005/008700 and US Patent No: 5981297 describes magnetoresistive detection strategies. US Patent Application No: 2005/0147981 describes a scanning probe microscopy (SPM) approach.

Xu and Lam (2003) also describe the use of Surface Plasmon Resonance spectroscopy (SPR) as a detection method. SPR is based on a change of refractive index occurring upon binding of an analyte to a surface.

The use of magnetic beads is described in US Patent Application No: 2004/0033627 and 2002/0081714. U.S. Patent Application No: 2002/0060565 describes a ferromagnetic based magnetisable bead detector. US Patent Application No: 2005/0100930 describes magnetic nanoparticles and magnetic detector arrays using a spin valve detector or a magnetic tunnel junction detector.

Perez et al. (Nature Biotechnology, 20: 816-820, 2002) describes the use of magnetic nanoparticles (MNP) to label DNA and detect this using NMR in solution. The principle of the detection method disclosed in this paper is a Magnetic Relaxation Switch (MRS) system in which there is a transition from a dispersed state to an aggregated state in which the nanoparticles self-assemble in solution into larger nano-assemblies. The self-assembly of these nanoparticles is the switching of state that is detected by the NMR. Thus, the principle is that a binding or hybridization reaction changes the spatial distribution of MNPs such that high localized concentrations of MNPs occur in solution.

Wang et al. (US2005/0100930) relates to magnetic nanoparticles (MNP) and the use of MNP labelling and formation of surface immobilised arrays. In this application, the MNPs are used in much the same way as fluorescent labels and are attached to nucleic acid molecules which are then captured by a complementary sequence attached to a detector, such as a spin valve detector or a magnetic tunnel junction (MTJ) detector. In this application, it is the attachment of a MNP labelled molecule to the immobilised probe that provides a detectable change in signal. The spin-valve and MTJ detector arrays are specially constructed substrates (non-magnetic and ferromagnetic layers and various magnetic layers), which detect the attachment of MNP's themselves, in effect by determining the change in electrical resistance caused when the MNPs associated with the sample bind to the probes at given locations on the detector.

Jain et al (US2002/0081714) discloses methods of forming random microarrays using magnetic beads or particles to which probes may be attached. The beads are detected by trapping them in regions of localised magnetic field and then detecting the change in local magnetic bead concentration that occurs once a bead has been trapped. Thus, the role of the MNPs in this application is to position the MNPs and detection of binding to probes is carried out using conventional techniques. Similar techniques are disclosed in US 2003/092029.

Aytur et al. (US2004/0033627) discloses a sensor that uses magnetic beads to detect substances of interest. The method involves the use of a small-scale Hall-Effect detector (HD) to detect the presence of the magnetic bead.

### Summary of the Invention

Broadly, the present invention concerns methods of detecting the interaction of target substances present in fluids with an array comprising one or more probes present or locatable, e.g. in the case of a bead array, on a substrate at one or more locations. The method is based on detecting the changes in the NMR signal arising from spin-carrying molecules.present in a fluid in the vicinity of the probes that occur when target substances interact with probes in the array. This provides a combinatorial method of assessing such interactions and the capability of using this approach for imaging and/or signal quantification. By way of example, the NMR. signal changes induced by such molecular and biomolecular interactions may be due.to changes in the NMR relaxation rates, T₁, T₂ or T₂^{*}, of the fluid molecules present or introduced into the direct vicinity of the immobilised probe, or may be due to changes in other NMR signals such as chemical shift.

Thus, the principle of operation of the present invention differs from prior art approaches since it employs changes in the NMR signal from the vicinity of the probe to assess molecular and biomolecular interactions across a microarray when target substances interact with the surface immobilised probes. This differs from the approach used in Perez et al. as the detection of the interactions take place at an array surface, rather than depending on the detection of a disperse-to-aggregated transition taking place in solution. In fact, Perez et al. do not envisage any change in NMR signal from the MNPs to be detectable in the absence of the occurrence of their solution phase transition.

The present invention also differs from the approaches disclosed in Wang et al. (US2005/0100930) in which magnetic nanoparticles are used as labels to produce a change in electrical resistance caused when target substances bind to substrates with magnetic layers such as spin-valve and MTJ detectors. The use of magnetic layers in the substrate in Wang et al. would also have the effect of preventing the NMR based detection techniques from working.

Furthermore, the present invention differs from the general use of magnetic nanoparticles as labels. In the prior art, MNPs are detected directly as an indication of the presence of a target substance, whereas in embodiments of the present invention that employ MNPs, they are used as magnetic contrast agents, i.e. as components for introducing a localised magnetic field for modulating the NMR signal produced by the spin-carrying species in the vicinity of the probes with which the target substance interacts or binds. The modulation may be either to amplify or reduce the NMR signal to thereby provide a pos.itive or negative contrast. Thus, the use of MNPs in some embodiments of the invention may help to overcome the problem that current NMR microscopes may not be sufficiently sensitive to detect the change in the relaxation of the spin-carrying NMR species in the fluid on the array unless an amplification technique is used. The role of the MNP is therefore to amplify the change in NMR signal to a level that is measurable.

Accordingly in a first aspect, the present invention provides a method of detecting an interaction of one or more target substances present in a fluid on a microarray, the method employing:
a microarray comprising a plurality of probes immobilised or immobilisable on a substrate at a plurality of locations, wherein the probes are capable of interacting with one or more target substances that may be present in the fluid, the method comprising:
   contacting the probes with the fluid under conditions in which one or more of the target substances, if present, interact with their respective probes, wherein the fluid comprises spin-carrying molecules detectable by nuclear magnetic resonance (NMR);
   where the probes are immobilisable on the substrate, immobilising the probes at locations on the microarray; and
   measuring a change in a NMR signal originating from the spin-carrying molecules in the vicinity of the probes at one or more locations in the microarray to determine whether the interaction between a target substance and its respective probes has occurred.

When the probes and/or the one or more target substances are associated with magnetic nanoparticles, a change in a NMR signal originating from the spin-carrying molecules in the vicinity of the probes at one or more locations in the microarray is measured to determine whether the interaction between a target substance and its respective probes has occurred, wherein the magnetic nanoparticles associated with the probes and/or one or more target substances amplify the change in the NMR signal by introducing a localised magnetic field gradient when the one or more target substances interact with their respective probes, thereby amplifying the NMR relaxation rate of the spin carrying molecules by enhancing their dephasing or causing a loss of coherence of their nuclear spins.

When neither the probes nor the one or more target substances are associated with a magnetic component, a change in a NMR signal originating from the spin-carrying molecules in the vicinity of the probes at one or more locations in the microarray is measured to determine whether the interaction between a target substance and its respective probes has occurred, but in this embodiment the change in NMR signal is caused or amplified by one or both of
(i) a change the diffusion constant of the spin-carrying molecules due to the interaction of the probes with the one or more target substances; and
(ii) an increase in the surface-enhanced relaxation of the spin-carrying molecules due to the interaction of the probes with the one or more target substances (T1 and T2 changes)

When the change in NMR signal is caused or amplified by (i), the method comprises amplifying the change in the NMR signal by applying an external magnetic field gradient during the measuring step to amplify changes in the NMP relaxation rate caused by the interaction of the probes and target substances affecting the diffusion constant of spin-carrying molecules in the fluid sample.

The interactions that may be detected or monitored using the methods described herein include binding, hybridisation, absorption, cross-linking and/or adsorption.

The present invention is based on the realisation that the interaction of surface bound probes and target substances on a microarray will influence the NMR signal arising from the spin-carrying molecules, e.g. protons in water or another fluid in which the molecules of the fluid comprise atoms that are NMR active, when the array is exposed to the fluid and the interaction between target substances and probes takes place.

Accordingly, in the present invention, the interaction or binding of target substances to their respective probes causes changes in diffusion constant of the spin-carrying molecules in the vicinity of the probes, thereby altering the NMR signal of the spin-carrying molecules that is detectable in the presence of an externally applied magnetic field gradient during the measuring step. In some embodiments, the probes or target substances may be associated with a component having a structure that enhances the change in diffusion constant to amplify changes in the NMR relaxation rate. By way of example, the component may have a dendritic or polymeric structure.

Alternatively or additionally, the target substances may be associated with a component that introduces a localised magnetic field gradient when the target substances interact with or bind to their respective probes, thereby altering the NMR signal of the spin-carrying molecules by enhancing their dephasing or causing a loss of coherence of the nuclear spins (T₂^{*} change). A preferred component for introducing a localised magnetic field gradient is a magnetic contrast agent such as a magnetic nanoparticle (MNP). However, instead of measuring the magnetic properties of the MNP itself, the method employs the localised magnetic field provided by the MNP to modulate (e.g. to amplify or reduce) the signal of the spin-carrying molecules of the NMR fluid.

Alternatively or additionally, the interaction or binding of target substances to their respective probes may cause surface-enhanced relaxation of the spin-carrying molecules in the fluid (T₁ and T₂ changes), e.g. where the appearance or the disappearance of nanoscale surfaces around a MNP label (or labelled probe or target) on which surface-enhanced relaxation of the spin-carrying molecule in the NMR fluid can occur (T₁ and T₂ changes). This will cause changes in the detected NMR signal without the need for any externally applied magnetic field gradient. By way of clarification, a MNP labelled probe or target in the system will cause surface-enhanced relaxation to the spin-carrying molecules in the NMR fluid, as these molecules will frequently collide with these surfaces due to self-diffusion. The transition between the non-interacting and interacting states of probe and target can be made to reveal or to hinder these nano-surfaces, and if a substantial amount of these small surfaces are altered, a detectable change in the NMR signal will be generated. An example of such a nano-surface would be to have a coated MNP so that the molecular collision with the coating rather than directly with the MNP ensures that the relaxation times are unchanged.

These mechanisms for inducing changes in the NMR signal of the fluid described above may occur independently or mutually of each other, or a further mechanism that contributes to a change in the NMR signal.

The methods disclosed herein may also have advantages over conventional detection techniques used with microarrays, and in particular the widespread use of fluorescent labelling. For examples, in spotted microarrays, the spots can have irregular shapes, non-uniform sizes and non-uniform immobilisation of the probes. Furthermore, these arrays are typically formed in impermeable substrates because while immobilization of the probes on porous substrates is possible, it can interfere with measurement of fluorescence. Fluorescent labelling of targets can suffer from dust contamination providing spurious contributions to fluorescent signals. In addition, the use of fluorescent labels is also sensitive to non-specific adsorption of labelled targets which provides a high level of background. Fluorescent labelling also has difficulty with samples/substrates that are opaque. Porous substrates include those which are uniformly porous and substrates comprising a porous membrane/layer on a substrate.
Examples of porous substrates include glass frits, sintered glass, a porous polymer matrix etc. The use of porous substrates in conjunction with MNPs may help to enhance the sensitivity of the NMR measurement, by allowing more magnetic nanoparticles to be present in the sensitive volume. It would also allow greater control of spot shape, size and homogeneity by control of substrate properties than.is possible on a simple solid substrate.

In comparison, the use of NMR based detection offers a number of further advantages over conventional detection techniques. NMR is a widely used technique, which should provide spot signals that are robust against the exact shape, size and uniformity of immobilised spots. NMR is also flexible due to the ability to tailor the relaxation time measurements, pulse sequences and use of a range of NMR carrying fluids, in addition to water, to optimise NMR contrast. For example, a wide range of natural and synthetic oils carry excellent NMR signal, as well as numerous fluorinated gases. As regards gas NMR, hyperpolarisation in combination with NMR is a well-established technique that provides signal strength that is much larger than that of liquid water NMR, and could be used in accordance with the present invention.

In embodiments of the invention that use MNPs, the signal changes caused by the presence of MNPs are essentially due to the spin-carrying molecules experiencing self-diffusion in magnetic field gradients in the vicinity of the magnetic nanoparticles, whilst the signal is being collected, normally within a time duration of a few to a few hundreds of milliseconds. Different fluids will have different diffusion constants making the technique flexible in its implementation and, for example, allowing the size of MNPs to be matched to a fluid with a given diffusion constant for optimised sensitivity.

In embodiments of the invention that employ NMR-microscopy, using externally applied field gradients will allow the rapid collection of signal across wide spatial areas, and decoded into localised signals using Fourier transformation using methods well known and proven in the art. Combining a raster scanning and field gradient method would allow a balance between time needed to complete array scanning and the image resolution.

In some embodiments, the methods disclosed herein could use a range of liquids and/or gases for imaging to provide multiple data sets or measurements to be made. This may have the advantage of improving signal-to-noise ratio and multiple confirmation for identifying probe-target substance interaction and level of interaction, thereby helping to increase confidence in conclusions drawn from a microarray experiment. As illustrated in the examples, the fluid may be chosen in view of its wetting properties as the use of water for the imaging fluid can be hampered by the hydrophobicity of the surface of the substrate. Accordingly, in some circumstances it may be preferable to chose an imaging fluid that provides the combination of NMR signal and has satisfactory wetting properties for imaging a spot on a given type of substrate.

In contrast to some prior art techniques, the use of the NMR based signal detection is fully compatible with the use of a wide range of substrates on which the array is pre-immobilised or formed *in situ*. This includes the use of substrates comprising a porous membrane which can hinder signal capture when fluorescent labels are used. The possibility of using porous substrates provides flexibility to design substrates to help to prevent other problems that can occur when non-porous substrates are used. By way of illustration, this may help to prevent donut formation that occurs when robotic spotting onto glass substrates. The use of porous materials (e.g. inkjet paper) would also enable substrates to be designed that could take advantages of techniques to control spot size, shape and homogeneity that are well-developed in the printing industry.

The methods of the present invention relying on exploiting changes in relaxation rates may be implemented using any sort of apparatus for carrying out pulsed or continuous nuclear magnetic resonance measurements. These include conventional NMR spectroscopy, magnetic resonance imaging (MRI), NMR microscopes and single-sided magnetic resonance imaging devices such as NMR-MOUSE® devices. The use of single-sided MRI devices for detecting interactions taking place on microarrays is a particularly convenient and inexpensive way of implementing the present invention because of the correspondence in dimension between microarrays and the planar outer detection surface of such devices.

The methods of the present invention relying on exploiting more subtle changes in the NMR signal may require the need for being implemented on the more sophisticated types of NMR instruments, benefiting of highly homogeneous polarizing field and/or high field strength.

Accordingly, in a further aspect, the present invention provides a method of detecting the interaction of one or more target substances present in a fluid and a microarray comprising a plurality of probes present on a substrate at a plurality of respective locations that are capable of interacting with one or more target substances that may be present in the fluid, the method comprising measuring a signal from a spin carrying species associated with one or more of a target substance, a probe or the fluid using a single-sided magnetic resonance imaging device such as a NMR-MOUSE^{®} device.

The use of magnetic contrast agents on both probe and target substance may enable signal from non-specific adsorption to be eliminated or reduced. A range of magnetic nanoparticle contrast agents have already been developed for *in vivo* and *in vitro* applications of MRI and have been shown to work with substances such as DNA and proteins. Whilst medical MRI is often expensive, NMR-microscopy using an instrument such as the NMR-MOUSE^{®} is relatively inexpensive and continuous wave NMR (CWNMR) hardware is even less expensive.

Embodiments of the present invention will now be described by way of example and not limitation with reference to the accompanying figures and examples.

### Brief Description of the Figures

**Figure 1** shows the concept of a grid/microarray of immobilised probes (P₁, P₂, ...), where the probes may or may not be labelled with magnetic nanoparticles.
**Figure 2** shows how magnetic labelling of probe, target or both probe and target results in NMR contrast when a probe P1 interacts with a target T. a) Magnetic nanoparticle (MNP) attached to target, b) MNP attached to probe, and c) MNP attached to both probe and target. In each case the NMR signal may be measured after a wash step and using a range of NMR fluids. Relative positions of MNP to probe, target or substrate is indicative only. It is likely that a linker for the MNPs will be needed.
**Figure 3** shows examples of linking strategies for probes/targets/substrates to MNPs.
**Figure 4** shows a side profile view of NMR imaging of an array based on uniform magnetic field without field gradients. Each spot is brought within the NMR sensitive volume in turn. There is no requirement for externally applied magnetic field gradients if MNPs are used.
**Figure 5** shows how field gradients may be incorporated to obtain slice selection and/or lateral selection to create images and/or to measure changes in diffusion constants. Non-linear field gradients could also be used, although NMR data would then require more sophisticated processing.
**Figure 6** shows imaging of an array. The entire array may be imaged using a field gradients approach or a subset of spots may be imaged using field gradients and then the centre of the region being imaged moved by a relative motion of substrate and the NMR apparatus.
**Figure 7 to 9** show the NMR signal acquired in the experiments described in Examples 3.1, 3.2 and 3.3.

### Detailed Description

### General introduction

The present invention relates to a method of imaging and quantitatively measuring biomolecular and molecular interactions on arrays (e.g. nucleic acid, protein, cell or chemical microarrays) using an alternative method to the use of fluorescent labels and confocal microscopy or other existing labelling methods. The concept is to use nuclear magnetic resonance (NMR) or NMR microscopy, which is magnetic resonance imaging (MRI) applied to the small scale, to detect changes in the NMR signal of a spin-carrying component of a fluid in contact with a microarray. These changes are caused by the interaction or binding of target substances to probes. Several mechanisms, acting independently or simultaneously, are possible including,
(i) the diffusion coefficient of the nuclear spin-carrying molecules in the vicinity of the target-substance probe complexes, can be altered by the interaction of interest. This effect can in turn be detected, inter alia, by the application of an externally applied magnetic field gradient, or by the association of the probes or target substances with components such as super-paramagnetic iron oxide (SPIO) or other magnetic nanoparticles or NMR contrast agents;
(ii) the area or number of areas upon which nuclear spin-carrying molecules may experience wall-enhanced relaxation,
(iii) the appearance or disappearance of local field gradients due to MNPs, i.e. so that there would be no need here to have any change in diffusion constant.

This effect can in turn be amplified, *inter alia,* by the application of an externally appplied magnetic field, or the association of the probes or target substances with components such as super-paramagnetic iron oxide (SPIO) or other magnetic nanoparticles as NMR contrast agents.

### Arrays

The method of the present invention are applicable to all types of arrays or microarrays including spotted microarrays, lithographic microarrays or bead microarrays, and are applicable to the 2D arrays generally used in the art as well as the 3D arrays. By way of example, the probes are preferably capable of specifically binding one or more target substance. Examples of probes include nucleic acid molecules, proteins, peptides, cells or chemical compounds. Nucleic acid probes may be DNA, oligonucleotide, mRNA or cDNA.

Commonly in the art, microarrays are with oligomer arrays, and more especially oligonucleotide or peptide arrays. As is well known in the art, nucleic acid is a polymer or oligomer of pyrimidine (U, C, T), or purine (A, G) nucleotides. In the present invention, the terms "oligonucleotide" and "nucleic acid" are used interchangeably. Typically, the oligonucleotides synthesised on the substrate are at least 10 nucleotides in length, more preferably at least 20 or 25 nucleotides in length to provide satisfactory hybridisation and discrimination when binding a target nucleic acid sequence. The oligonucleotides and nucleic acid molecules used in the present invention may be formed from naturally occurring nucleotides, for example forming deoxyribonucleic acid (DNA) or ribonucleic acid (RNA) molecules. Alternatively, the naturally occurring oligonucleotides may include structural modifications to alter their properties, such as in peptide nucleic acids (PNA) or in locked nucleic acids (LNA). For example, the improved hybridisation properties of these modified oligonucleotides can be used to reduce the length required for their use in arrays. The solid phase synthesis of oligonucleotides and nucleic acid molecules with naturally occurring or artificial bases is well known in the art. The arrays of oligonucleotides, formed from natural or artificial bases may be synthesised in either the 3' → 5' or 5' → 3' directions. The present invention can also use to peptide oligomer arrays formed from sequences of amino acids, or similar compounds, for example L-amino acids, D-amino acids, or synthetic amino acids.

As used in the present invention, "array" or "microarray" includes a group of molecules (the probes) intentionally created and arranged on a substrate at a plurality of array elements or locations or which are formed on a substrate, e.g. in the manner of a bead array. It is generally preferred that there is a substantially homogeneous population of molecules at each element of the array, either through synthesis of the probes at predetermined locations in the array or in the case of a bead array because each location generally accommodates a single bead. The molecules present in the array may be prepared either synthetically or biosynthetically and may be identical or different from the molecules present at other elements of the array. There is a wide variety of substrates on which arrays may be laid down. Similarly, the geometry of the elements forming the array may be varied: However, for convenience, it is typical in the art to employ a substrate, typically formed from an inorganic material such as glass or a plastic material (e.g. nylon, polyethylene) resistant to the organic solvents used in reagent solutions, and on which the molecules at each element of the array can be laid down as a grid of squares, rectangles or circles. Preferably, substrates have rigid or semi-rigid surfaces and, although it is generally preferred that at least one surface of the substrate is substantially flat, it is known in the art to separate elements of the array by using surface features, such as ridges or grids or coatings.

### NMR, MRI and NMR Microscopy

Nuclear magnetic resonance (NMR) is based upon the intrinsic magnetic moment of an atom's nucleus. NMR uses a magnetic nucleus, such as that of a hydrogen atom, by aligning it to an external magnetic field and perturbing this alignment using an electromagnetic field. The response of the nuclei to the field is what is measured in nuclear magnetic resonance spectroscopy and magnetic resonance imaging.

Magnetic resonance imaging (MRI) is well-known for its ability to image the brain, lungs, etc. The method uses magnetic field gradients to determine the NMR signal from localised volumes of a sample. Since the Larmor precession frequency depends upon the local magnetic field, a spatially varying magnetic field superimposed on a static magnetic field introduces a correspondence between resonant frequency and spatial location. In particular, a complete three-dimensional image can be reconstructed based on the frequency analysis of a collected NMR signal. MRI offers potential for complex information via the measurement of the T₁ and T₂ (or T₂^{*}) relaxation times. MRI is now a well-established medical and materials imaging technique. In NMR microscopy, an image is obtained of a slice tens to hundreds of microns thick with one micron to tens of micron scale lateral resolution. Magnetic resonance (MR) microscopy is defined as MR imaging where sub-millimetric spatial resolution is achieved. Using one gradient coil, one could successfully spatially identify a succession of probes that were to be placed along a line matching the direction of the gradient, although the MR signal would be averaged in the two other directions. Using two gradient coils, one could successfully spatially identify a 2D array of probes, although the MR signal would be averaged in the vertical direction. Using three gradient coils, the vertical direction could be spatially resolved, or one could successfully spatially discriminate a stack of 2D array of probes.

There are a range of NMR microscope instruments with specific performance. These instruments traditionally are cylindrical in shape with an internal hollow tube, horizontal or vertical, where the sample to be imaged/measured is placed. The cylindrical hardware typically consists of an outer superconductive magnet, in which the gradient coil is inserted, in which a radiofrequency coil is then placed for further accommodating the sample itself. Because of issues regarding the sensitivity of NMR signal detection, the inner diameter of the tube welcoming the sample has to be as close as possible to the outer dimension of the sample being investigated. This limits the microscopy to samples below an overall size of a few centimetres. Recently single sided magnetic resonance imaging apparatus have been developed. In the NMR-MOUSE^{®}, imaging of -samples placed above its outer planar surface is achieved (i.e. there is no need to place a sample within a tube). Moreover, the instrument does not require a superconducting magnet and has a relatively low cost. In some embodiments of the present invention, the use of NMR-MOUSE^{®} devices in the methods disclosed herein provides a particularly convenient way of detecting the NMR signal changes caused by changes in the relaxation rate or diffusion coefficient when target substances bind to the array.

NMR microscopy is described by Aguayo et al., Nuclear-Magnetic-Resonance Imaging Of A Single Cell, Nature, 322: 190-191 (1986). High resolution NMR microscopy is described by Lee et al., One micrometer resolution NMR microscopy, J. Mag. Res. 150: 207-213 (2001).

A sample mount for NMR microscopy and NMR spectroscopy that enables a specimen to be accurately placed within an NMR apparatus is described in US Patent No: 5416414. A cryogenic probe for NMR microscopy is described in US Patent No: 5258710.

A low-cost single sided magnetic resonance imaging apparatus (the NMR-MOUSE^{®}) is described in US Patent No: 6977503, 6489767 and US Patent Applications No: 2005/0040823, 2002/0089330, 2002/2079891. This device is particularly useful for carrying out the methods disclosed here as the detection volume is well matched to the dimensions of microarrays.

### Magnetic Contrast Agents

It is common practice in MRI to improve the image by using a magnetic contrast agent. Both the spin-lattice relaxation time, T₁, and spin-spin relaxation time, T₂, of protons in water or other fluids may be altered in the presence of paramagnetic species. Common contrast agents are magnetite, maghemite, monocrystalline iron oxide nanoparticles, superparamagnetic iron oxide (SPIO) and Gadolinium (Gd) based compounds.

SPIOs allow contrast of an area of interest to be improved by increasing both relaxation rates of protons in water or other fluids which include NMR gases and hyperpolarized gases. SPIO contrast agents are often coated with sugars or silicates to prevent aggregation, to render them water soluble and to provide functionalization for the conjugation of biomolecules. Sizes from several to several hundred nanometers and batches with very monodisperse sizes can be created. SPIOs are most often hematite/maghemite/magnetite (Fe₂O₃/Fe₃O₄), and consist of a crystal with a single magnetic domain. Magnetic nanoparticles are efficient as relaxation promoters, and their effect on the relaxivities of water is measurable even at nanomolar concentrations.

The use of ferromagnetic and superparamagnetic nanoparticles as contrast agents can induce a more than ten-fold increase in proton relaxivities (Coroiu, Relaxivities of different superparamagnetic particles for application in NMR tomography, J. Magnetism Magnetic Maters. 201, 449-452 (1999)).

Synthesis, characterisation and efficiency in MR imaging of SPIO and ultrasmall SPIO, including the relationship between SPIO concentration and relaxation times and relaxivities, is described by Lawaczeck et al., "Superparamagnetic iron oxide particles: contrast media for magnetic resonance imaging", Appl. Organometal. Chem. 18, 506-513 (2004).

### Molecularly Targeted Imaging

Nanoparticle probes that may be employed in accordance with the present invention can be created with specific sizes, coating thickness, surface chemistry, and targeting ligands and can be used to target specific organs, cells and molecular markers. Examples include:

The targeting of specific proteins and nucleic acids, see for example Nitin et al., "Functionalization and peptide-based delivery of magnetic nanoparticles as an intracellular MRI contrast agent", J. Biol. Inorg. Chem. 9(6): 706-712 (2004).

SPIOs conjugated to monoclonal antibodies, see for example Artemov et al, "MR molecular imaging of the Her-2/neu receptor in breast cancer cells using targeted iron oxide nanoparticles", Mag. Res. Med. 49 (3): 403-408 (2003) and Ahrens et al., "Receptor-mediated endocytosis of iron-oxide particles provides efficient labelling of dendritic cells for in vivo MR imaging", Mag. Res. Med., 49 (6): 1006-1013 (2003).

Conjugation of short oligonucleotides to the surface of SPIOs and subsequent hybridization of these particles to complementary oligonucleotides, see for example Perez et al., "DNA-based magnetic nanoparticle assembly acts as a magnetic relaxation nanoswitch allowing screening of DNA-cleaving agents", J. Am. Chem. Soc. 124 (12): 2856-2857 (2002).

Conjugation of SPIO particles to a peptide sequence, see for example Josephson et al., "High-efficiency intracellular magnetic labelling with novel superparamagnetic-tat peptide conjugates", Bioconjugate Chem. 10(2): 186-191 (1999) and Dodd et al., "Normal T-cell response and in vivo magnetic resonance imaging of T cells loaded with HIV transactivator-peptide-derived superparamagnetic nanoparticles", J. Immunol. Methods 256 (1-2) : 89-105 (2001).

Conjugation of SPIO particles to transfection agents, see for example Arbab et al., "Efficient magnetic cell labeling with protamine sulfate complexed to ferumoxides for cellular MRI", Blood, 104(4): 1217-1223 (2004).

Inclusion of SPIO into liposomes which can further be made to be targeted, see for example Martina et al., "Generation of superparamagnetic liposomes revealed as highly efficient MRI contrast agents for in vivo imaging", J. Am. Chem. Soc., 127(30): 10676-10685 (2005).

The use of monocrystalline iron oxide particles for studying biological tissues is described in US Patent No: 5492814. MRI contrast agents comprising a paramagnetic metal ion bound to a complex for the detection of physiological agents are described in US Patents Nos: 5980862 and 6713045.

DNA-dependent MRI contrast agents are described in US Patent Application No: 2005/0112064.

The magnetic contrast agents such as MNPs may be combined with the use of other labels such as fluorescent labels so that both fluorescent imaging and NMR imaging of the interaction between the probes and target substances on the microarrays is possible.

The MNPs may also be loaded into liposomes, which themselves have probes (e.g. strands of DNA) attached to them, and to immobilize the liposomes onto a substrate.

### Experimental

As will be apparent from the discussion provided above, the present invention relates to a NMR detection of the interaction or binding of target substances to microarrays. The principle of operation of the present invention relies on the localised NMR signal of a spin-carrying species in the proximity of probes forming a microarray being changed when target substances interact with or bind to the probes.

In one embodiment using magnetic nanoparticle contrast to assess molecular and biomolecular interactions across a microarray, as targets interact with surface immobilised probes. This provides a combinatorial method of assessing such interactions and provides the ability for both imaging and signal quantification.

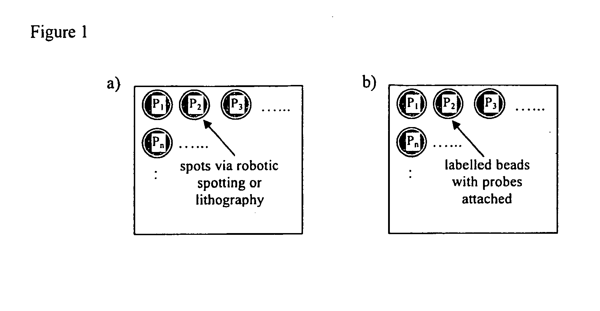

Figure 1 shows schematically how an array of probes may be formed for use in accordance with the present invention. The methods of interrogating arrays disclosed herein are applicable for use with arrays formed with any of the types of probes used in the art, such as nucleic acid molecules, antibodies, proteins, peptides, cells and/or chemical probes. Figure 1a shows an approach in which an array is preformed and the probes are localised as one or more spots or locations immobilised on a substrate in a regular or irregular fashion, for example by spotting or lithographic synthesis, thereby allowing the probes present at a given location in the array to be known from their position in the array. The present invention may also be used in conjunction with arrays that are formed in use, for example where the probes are immobilised on beads which become located on the substrate after interaction with a sample containing target substances. In this case, the probe present at a location may be determined using a label to allow decoding of the probe type (Figure 1b).

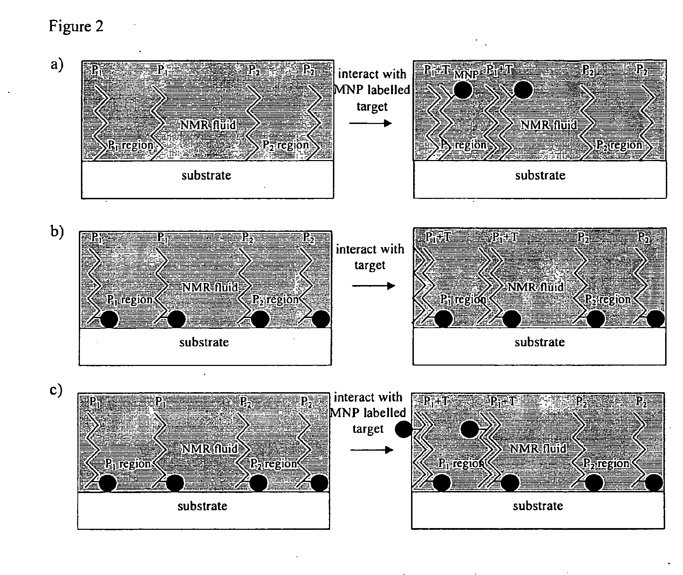

Figure 2 shows three approaches for amplifying the changes in the NMR signal caused by changes in the relaxation rate of the spin-carrying molecules in the fluid in the vicinity of the probes when the probes bind to target substances. These changes in the NMR signal arise because the probe-target substance interaction modulates one or more of (i) the diffusion coefficient of the nuclear spin-carrying molecules in the vicinity of the target-substance probe complexes, (ii) the area or number of areas upon which nuclear spin-carrying molecules may experience wall-enhanced relaxation, (iii) the appearance or disappearance of local field gradients due to MNPs. In Figure 2, P₁ and P₂ represent the probes present at two locations of a microarray formed on a substrate. The microarray is contacted with by a fluid sample comprising target substances T that are capable of specifically binding to the probes at one or more of the locations. At some point, a spin-carrying molecule detectable by NMR is introduced into the system to permit detection of the interactions between probes and target substances by NMR. The spin-carrying molecule may be initially present in the fluid sample, added to it simultaneously with or subsequent to contact with the array and/or the spin carrying molecule may be present in a further fluid that replaces the fluid sample used in the initially contacting step. In this embodiment of the invention, the magnetic nanoparticles MNPs, which include either any sub-micron scale particle that spatially distorts the.local magnetic field or any single magnetic domain carrying particle, are used either with the probe (P) or the target (T) or both (Figure 2 a-c). Since the NMR signal depends upon the magnetic nanoparticle interactions with fluid (e.g. water, another magnetic nuclei containing liquid, an NMR gas or a hyperpolarized gas) or the direct coupling of two magnetic nanoparticles, the transition between the non-interacting and interacting states of probe and target will generate a measurable change in the relaxation times of fluid molecules.

Alternatively or additionally, a detectable NMR signal may be obtained without using MNPs, for example by applying an external magnetic field to the microarray during NMR detection.

Thus, any reagent having a structure which substantially alters the diffusion constant of the NMR fluid in which it resides, can be used either with the probe (P) or the target (T) or both, as an alternative to using an MNP, provided an external magnetic field gradient is then used.

An example would be to use a micron-scale dendritic structure rather than the MNP shown in Figure 2a and to then perform NMR with an external magnetic field gradient applied. A variation on this would be to attach a dendritic or other polymeric structure (instead of the MNP) after the probe and target had interacted.

If a magnetic field gradient is externally applied to the probe under investigation, the NMR signal of the fluid in the vicinity of the probe (e.g. water, another magnetic nuclei containing liquid, an NMR gas or a hyperpolarized gas) will change with the diffusion constant, and the transition between the non-interacting and interacting states of probe and target will generate a measurable change in the relaxation times of water or other suitable fluid (including liquids, NMR gases and hyperpolarized gases).

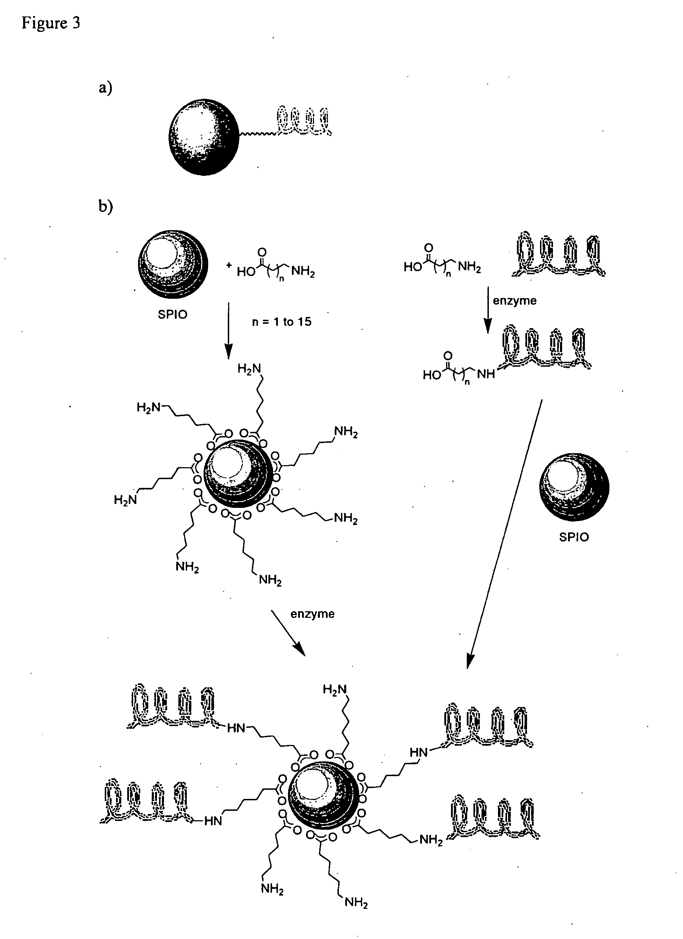

It will be recognised that linker chemistry may be employed to link probes, target substances or substrates to MNPs (Figure 3a-c). It is also possible for a single MNP may have more than one target attached to it (e.g. multiple cDNA strands, etc). Figure 3a shows a single probe/target linked to single MNP via linker/spacer and Figure 3b an example of a possible linker/spacer immobilised to an MNP that can be coupled to probe/targets/substrates (shown schematically as a helix) using an enzyme catalyst.

Spots may be created in a multitude of ways similar to existing microarray methods (lithographically, robotic spotting, arrangement of beads containing probes onto a surface, etc). In the preferred embodiment of the method a target is introduced (in a solution) and interactions allowed to complete (e.g. DNA hybridisation which could be followed by a washing step to remove non-specifically bound target material) before an NMR carrying fluid (a suitable liquid or gas) for measuring NMR contrast is introduced at a subsequent stage.

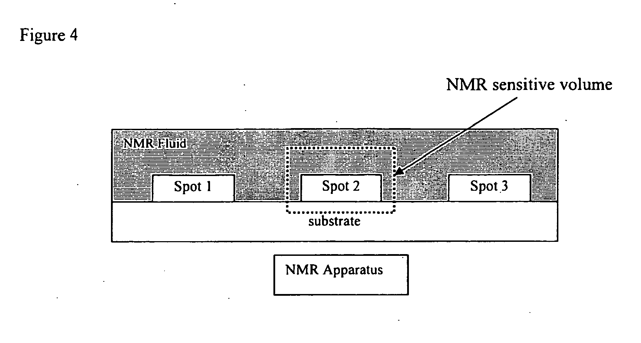

In its simplest form NMR imaging of the array may be achieved by ensuring each spot is brought within the volume of sensitivity of the NMR system whilst other spots remain outside (Figure 4). No magnetic field gradient is needed in this particular case and the image consists of a single NMR signal representing each spot. Either a substrate containing the spots may be moved to within the NMR sensitive region or the NMR system/sensitive volume may be moved across the array.

The NMR system may include either pulsed NMR or continuous wave NMR (CWNMR).

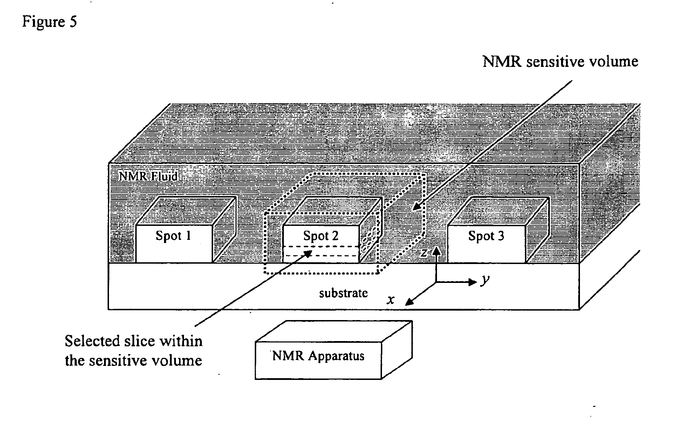

Figure 5 shows how field gradients may be incorporated to obtain slice selection and/or lateral selection to create images. The plane within which samples lie is called (x,y) and we call z, the third orthogonal axis. The field gradients consist of a magnetic field along the direction of the polarising field, for example the y-axis. With one of these field gradients, say the one proportional to the z axis, as in B(r)= G_{z}z j, a slice within the sensitive area may be selected (dotted lines on the figure), or a profile of the sample along the z direction may be measured. By using a combination of two field gradients, the one proportional to the x axis and the one proportional to the y axis, a two dimensional image of an array of samples can be imaged, if they lie within the sensitive volume of the instrument. Non-linear field gradients could also be used, although NMR data would then require more sophisticated processing. The array of samples can thus be measured without the need of moving the instrument relative to the samples. Thus, the present invention may employ one, two or three orthogonal linear magnetic field gradients to spatially encode the NMR signal coming from the volume of the sample that lies in the sensitive area of the instrument. A further embodiment is to use lateral field gradients to obtain a two-dimensional NMR image of a spot and/or to obtain images of multiple spots.

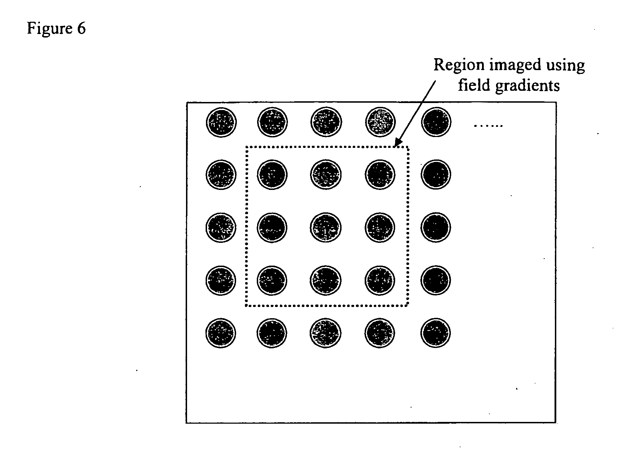

When imaging multiple spots it would be possible to use lateral field gradients alone to image a complete array, without the need of moving the array relative to the NMR instrument. The same gradients could also be used to spatially resolve the signal coming from a single spot, which could be on an array but not necessarily. However, it would also be possible to use a scanning motion of the substrate or NMR apparatus to image the whole array without recourse to lateral field gradients. A combination of a scanning motion of the substrate or NMR apparatus could be used in conjunction with field gradients to image the complete array and so optimise array imaging speed and image resolution (Figure 6). The system may be designed to be compatible with either unilateral (single-sided) or non-unilateral NMR microscope approaches.

As a further possibility, the methods of the present invention may be carried out using a range of different liquids and/or NMR gases (including hyperpolarized) to detect target substances binding to the probes and to thereby obtain a multiple set of NMR contrasts obtained for the same set of spots.

Alternatively or additionally, rather than the interaction between probes and target being allowed to complete and then a suitable NMR fluid (e.g. water, fluorinated or other NMR gases and hyperpolarized gases) being introduced to provide measurement of NMR contrast, the probe could be contained within a suitable liquid or gas for imaging (e.g. water) and the NMR signal monitored during the interaction period.

In a further embodiment for providing a method of quantifying molecular or biomolecular interactions, a calibrated NMR signal could be used which changes as a function of known nanoparticle concentration to further quantitate MNP concentration of spots with unknown concentration. In particular, 1/T₂ and 1/T₁ are known to be linearly related to nanoparticles concentration for a given fluid, nanoparticle size and over a broad range of concentrations.

### Examples

### Overview

Two types of NMR system have been used to confirm the ability of NMR and MRI to image magnetic nanoparticle based microarrays. In the first set of experiments a unilateral NMR profiling instrument was used to detect immobilization of a chemical species on glass surfaces. In the second set of experiments a non-unilateral, standard, cylindrical, horizontal small bore MRI instrument was used to image chemical arrays and oligonucleotide arrays.

### 1. Instrumentation

### a) Experiments using a unilateral NMR Instrument

In these experiments a profile NMR MOUSE^{®} [Perlo, J, Casanova, F., and Blumich, B. Profiles with microscopic resolution by single-sided NMR. J. Mag. Res. 2005. 176 (1): p 64-70] that collects the NMR signal coming from a thin and flat volume of sensitivity (approximately 200 µm x 20 mm x 20 mm) at 5 to 10 mm above the instrument was used. A strong (11.4 T/m) magnetic field gradient resides permanently across the selected slice. The presence of this gradient in conjunction with a suitable radiofrequency (rf) pulse sequence allows the collection of spin echoes that can be processed so as to obtain spatially resolved one dimensional profiles of the NMR relaxation rate present at a given position across the slice. [Goelman, G., and Prammer, M. G. The CPMG pulse sequence in strong magnetic-field gradients with applications to oil-well logging, J. Mag. Res. Ser. A. 1995. 113 (1): p. 11-18 MAR 1995]

The profile NMR MOUSE used, comprising of the polarising magnets assembly and the rf coil, has the shape of a parallelepiped rectangle (13 x 11 x 10 cm³). The NMR data was collected using a CPMG sequence (with multiple echoes and an accumulation of experiments) and the spin echoes Fourier transformed. NMR signal from regions of interest were obtained by moving the part of the sample of interest into the NMR sensitive area of the instrument.

### b) Experiments using a small bore MRI Instrument

In these experiments a Bruker^{®} 2.35 T small-bore MRI scanner with a radiofrequency coil of 72 mm internal diameter, proton-only resonator, was used to image both chemical and oligonucleotide arrays using RARE scan protocols. This allowed a complete set of images of a sample volume to be obtained without the need to physically move regions of interest into a particular NMR sensitive area. It also allowed three-dimensional images to be acquired and two-dimensional slices corresponding to a given array within those images to be extracted. All images were produced with Matlab^{®} from Fourier transforming the raw NMR signal collected by the scanner.

### 2. Materials and Surface Preparation Methods

### 2a) Synthesis of N- (3-triethoxysilylpropyl)-6-(N-maleimido)-hexanamide

*N*-Maleimidocaproic acid was prepared following the general literature procedure for the synthesis of *N*-substituted maleimide derivatives. Kalgutkar et al., Design, synthesis, and biochemical evaluation of N-substituted maleimides as inhibitors of prostaglandin endoperoxide synthases. J Med Chem, 1996. 39(8): p. 1692-703. In detail, 34 g of maleic anhydride was added to 45.5 g of 6-aminohexanoic acid and 700 mL of glacial acetic acid. The reaction mixture was stirred overnight and the resulting product was recrystallised as a white powder from IPA/H₂O.

The product (30g) was mixed with 125 mL of acetic anhydride and 6.15 g of sodium acetate and heated to 90°C for 2 hours. The reaction was then cooled and quenched with water. The aqueous solution was extracted with diethyl ether (3 x 40 mL) and organic extracts were dried over magnesium sulphate, filtered and concentrated under vacuum. The *N*-maleimidocaproic acid was then recrystallised from ethyl acetate as a white powder.

*N*-(3-triethoxysilylpropyl)-6-(N-maleimido)-hexanamide was prepared following a literature procedure. Choithani et al., N-(3-Triethoxysilylpropyl)-6-(N-maleimido)-hexanamide: An efficient heterobifunctional reagent for the construction of oligonucleotide microarrays. Anal Biochem, 2006. 357(2): p. 240-8. In detail, 13.5 g *N-*maleimidocaproic acid was added to 11 g of *N-*hydroxysuccinimide and 16 g of dicyclohexylcarbodiimide in tetrahydrofuran (25 mL) and was left to stir at room temperature for 3 hours. Following completion of the reaction, dicyclohexylurea was removed by filtration and to the filtrate 3-aminopropyltriethoxysilane (17.5 g) and triethylamine (8 g) were added. The reaction was left to stir for a further 6 hours at room temperature, concentrated under vacuum, dissolved in anhydrous benzene and filtered. The filtrate was concentrated under vacuum to yield *N*-(3-triethoxysilylpropyl)-6-(*N*-maleimido)-hexanamide.

### 2b) Design and preparation of oligonucleotides

Complementary 5' to 3' and 3' to 5' oligonucleotide sequences (5-CTCCTGAGGAGAAGGTCTGCTGGAC-3 and 5-GTCCAGCAGACCTTCTCCTCAGGAG-3) were modified with the incorporation of thiol (-SH) groups linked to the 5' end by a 6-carbon spacer (Sigma-Genosys) and subsequently reconstituted in water to a concentration of 100 µM. Trityl groups were removed by incubation with 0.04 M DDT in 0.17 M phosphate buffer (pH 8.0) at room temperature for 16 hours. DTT and thiol by- products were removed using NAP-10 columns, following the manufacturer's protocol (GE. healthcare). Finally the oligonucleotide fractions were verified by taking readings at 260 nm, pooled and diluted to stock aliquots of 60 µM.

### 2c) Preparation of glass slides and hybridization with oligonucleotides

New glass microscope slides (76 x 26 mm) were engraved with 12 circular spots (diameter 4 mm) in a 3 x 4 arrangement with separations of 3 mm using a Hobarts laser cutter linked to corelDRAW software. Slides were cleaned by being immersed in a methanolic solution of sodium hydroxide overnight, rinsed in distilled water, immersed in 37 % HCl for 2 hours, rinsed in water and stored in diethyl ether. (Choithani et al, supra). Following thorough drying, spots were coated with 10 µl of *N*-(3-triethoxysilylpropyl)-6-(*N-*maleimido)-hexanamide and irradiated for 3 x 15 seconds in an 800 W domestic microwave. Oligonucleotides (1 µl) were spotted onto coated slides which were then incubated at 37°C in a humidified chamber for 1 hour.

### 2d) Preparation of glass slides and hybridization with 4-aminothiophenol.

Both laser etched (template spot sizes from 0.5 to 5 mm in diameter) and virgin glass microscope slides along with cover slips (166 µm depth) where used as binding surfaces for the chemical arrays. An immobilised film of the aminothiophenol was prepared by suspending an acid washed glass microscope slide (as described above) length ways into a solution of the *N*-(3-triethoxysilylpropyl)-6-(*N-*maleimido)-hexanamide in DMF so that half of the slide was covered by the solution overnight. The excess solution was washed away with distilled water and the slide completely submerged into a solution of 4-aminothiophenol for two hours at 40°C. The excess 4-aminothiophenol was washed away with DMF and water before condensation with the SPIO bound carboxylic acid. These slides were used in Examples 3.1, 3.2 and 3.3.

In the case of the chemical arrays on etched glass microscope slides (76 x 26 mm) two templates were used: (i) 12 circular spots (diameter 4 mm) in a 3 x 4 arrangement with separations of 3 mm and (ii) 14 circular spots (7 with diameters of 0.5 mm on one line spaced 8 mm apart and 7 with diameters of 1.0 mm on a second line spaced 8 mm apart, the two lines were separated by 6.5 mm) in a 2 x 7 arrangement) *N*-(3-triethoxysilylpropy-1)-6-(N-maleimido) hexanamide was spotted onto the etched areas of the glass slides as described above for the oligonucleotides. 4-Aminothiophenol (0.05 M in benzene, 1 µl) was spotted onto coated slides which were then incubated at 37°C in a humidified chamber for 1 hour. These arrays were used in Examples 4.1 and 4.3. The array used in Example 4.2 had a template pattern of 3x4 with 4mm diameter spots and with DNA immobilised on the spots as described in section 2f.

### 2e.). Synthesis of dextran-coated superparamagnetic iron-oxide (SPIO) particles

Dextran coated SPIOs were produced using a similar method to one previously described in US Patent No: 5262176. Specifically, 7.01 g of iron(III)chloride hexahydrate (Sigma-Aldrich) and 100 g of dextran (*M*_{w} 9-llk) were added to 223 mL of distilled water, filtered (0.2 µm) and cooled to 4°C. This was added to 4.79 g of cooled and filtered iron(II)chloride tetrahydrate that was dissolved in 9.5 mL distilled water. The solution was rapidly stirred and neutralized by the drop-wise addition of 10 mL of ammonium hydroxide resulting in a dark green suspension. This was heated whilst stirring to 80°C for 1 hour and then maintained at this temperature for a further 75 minutes. The resulting colloid was filtered (0.2 µm) and separated from contaminants using a 20 mL size exclusion chromatography column packed with Superdex 75 (Amersham Biosciences). Dynamic light scattering (DLS) analyses showed 10-30 nm particulate size; larger aggregates (30-50 nm) can be removed by centrifugation (13000 RPM x 15 minutes).

### 2f) Cross-linking of SPIOs with epichlorohydrin, amination and attachment of oligonucleotides

Dextran coated SPIOs were cross-linked using the method described in US Patent No: 5262176. Epichlorohydrin (40 mL), 100 mL of 5M NaOH and 40 mL of distilled water were added to 20 mL of the colloid. The SPIOs were aminated by addition of 50 mL of concentrated ammonia to the above and heated to 37°C overnight with mixing. Josephson et al., High-efficiency intracellular magnetic labeling with novel superparamagnetic-Tat peptide conjugates. Bioconjug Chem, 1999. 10(2): p. 186-91. Epichlorohydrin was removed by dialyses (12-14 kD cut-off) with 20 changes of distilled water.

N-succinimidyl 3-(2-pyridyldithio) propionate (SPDP) (2 mL of a 50 µM solution in 25 mM DMSO) was added to 1.2 mL of aminated SPIO and 1.2 mL of 0.1 M phosphate buffer (pH 7.4) and left to stand at room temperature for an hour. (Josephson et al., supra). SPDP bound SPIOs (1.1 mL) (in 0.1 M phosphate buffer, pH 8.0) were incubated with thiolated oligonucleotides (550 µg) and the mixture was incubated overnight at room temperature. Josephson et al., Magnetic nanosensors for the detection of oligonucleotide sequences. Angewandte Chemie 2001. 40(17): p. 3204- 3206. SPIO bound oligonucleotides (15 mL) were added to glass slide bound oligonucleotides and left to hydrolyze in a humid chamber for 72 hours and washed with water immediately prior to analysis.

### 2g) Cross-linking of SPIOs with epichlorohydrin, amination and attachment of thiophenols.

Dextran coated SPIOs were cross- linked and aminated as described above for the SPIO-oligonucleotides.

Benzenethiol (1 g) or 4-mercaptobenzoic acid (1 g) in THF (1 mL) was added to 1.2 mL of aminated. SPIO and heated in a sealed vial in a domestic microwave (3 x 15 seconds). Unreacted benzenethiol / 4-mercaptobenzoic acid was removed by sublimation onto a liquid nitrogen cold finger (150°C, 10 mbar). The resulting SPIO bound benzenethiol-/ 4-mercaptobenzoic acid was dissolved into DMF (1 mL) and spotted (1.5 µL) onto the pre-prepared 4-aminothiophenol bound glass slides either directly onto the templated areas as in the retched glass microspope slides or as droplets of varying size onto the immobilised aminothiophenol filmed slide. The slides were then heated in a domestic microwave (3 x 15 sec), allowed to cool and washed with water and DMF.

### 3. Data from Experiments using a Unilateral NOR Instrument

### Example 3.1 Imaging of a single spot using SPIO particles and a droplet of water as the imaging fluid

Droplets of water were deposited as the imaging fluid onto the glass slide which had previously been prepared with a SPIO labelled region and a non-SPIO labelled region (see section 2d). A repetition time of TR = 100 ms was used to reveal the presence of SPIOs as a positive contrast. Five hundred averages were acquired, resulting in a 50 second duration experiment.

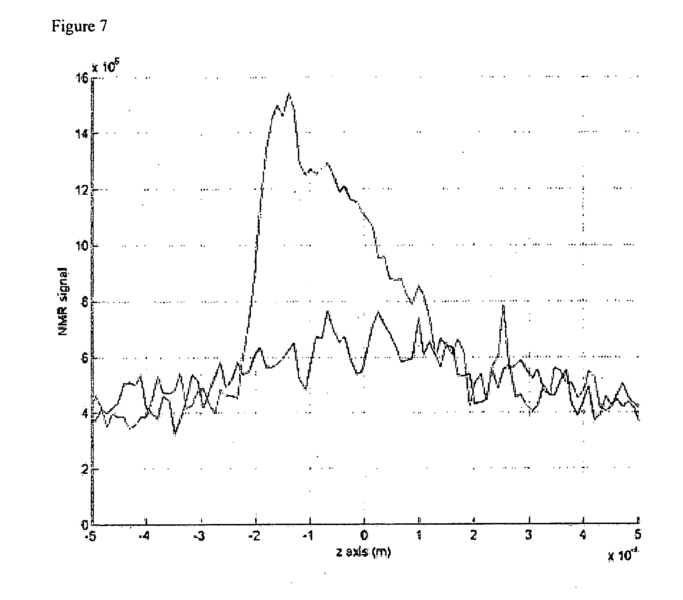

In Figure 7, the upper/red curve shows the signal acquired from a droplet deposited on area of the glass slide that had SPIO labelled region and the lower/blue curve is the NMR signal acquired using a droplet deposited on the side without a SPIO labelled region. The z-axis location of the peak in the upper curve corresponds to the plane of the surface of the glass slide at which the SPIO labelled region resides. The results show that a measurable difference as a positive contrast in SPIO labelled and non-SPIO labelled regions can be obtained using water as an imaging fluid. Modifying repetition time and number of signal averages would allow the NMR signal to be optimised.

One potential difficulty in using water for the imaging fluid is the possible hydrophobicity of the surface and its effect on the wetting of the surface by the water. The experiments were therefore repeated using droplets of ethanol, which wets this surface more readily, as the imaging fluid and a positive contrast was also observed. This demonstrates that imaging fluids may be chosen to have a combination of NMR signal and wetting properties for imaging a spot.

### Example 3.2 Imaging a multiple spots using SPIO particles and immersion in water as the imaging fluid

The glass dish prepared for example 3.1 with multiple SPIO-labelled and non-SPIO labelled spots was imaged by immersing the slide in water as the imaging fluid. Four different repetition times, TR, were used, 0.1 s, 0.5 s, 0.9 s and 1.3 s and one minute duration experiments were performed for each repetition time.

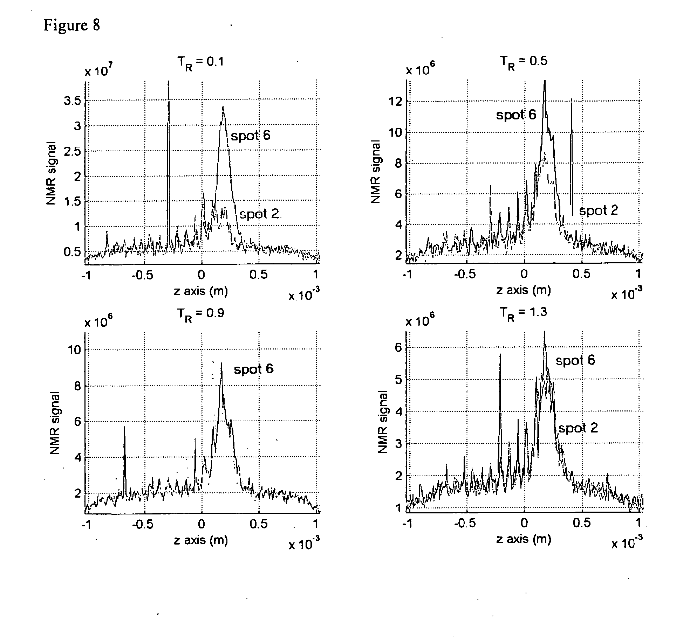

Figure 8 shows a comparison between NMR signal for spot 2, created using a 0.01 ml concentration of SPIO's, and spot 6, created using a 1 ml concentration of SPIO's. Spot 6 demonstrates a much higher signal, and the difference between the two signals decreases for increasing TR value, as expected. On the long TR value, the water content can be seen. This demonstrates that NMR signals related to the concentration of SPIO's within a region can be obtained.

By dividing the signal-to-noise ratio (SNR) of the short TR measurement by the SNR of the long TR measurement, an estimate of the concentrations of SPIO particles within a spot could be made. To increase the overall SNR longer duration measurements, for each TR value, could be performed. This type of measurement is based on T₁-relaxation effects.

### Example 3.3 Single spot reproducibility and repetition time dependence using water as the imaging fluid

Three slides produced in a single batch with two possessing a single SPIO-labelled spot and one possessing a blank spot were produced (see section 2d) and imaged. A small glans cover slip was placed on top of the 10 µL droplet of water on each slide, to spread and sandwich the water above the samples. NMR data was acquired using a range of (short) TR values: TR = 100, 200, 300 and 400 ms.

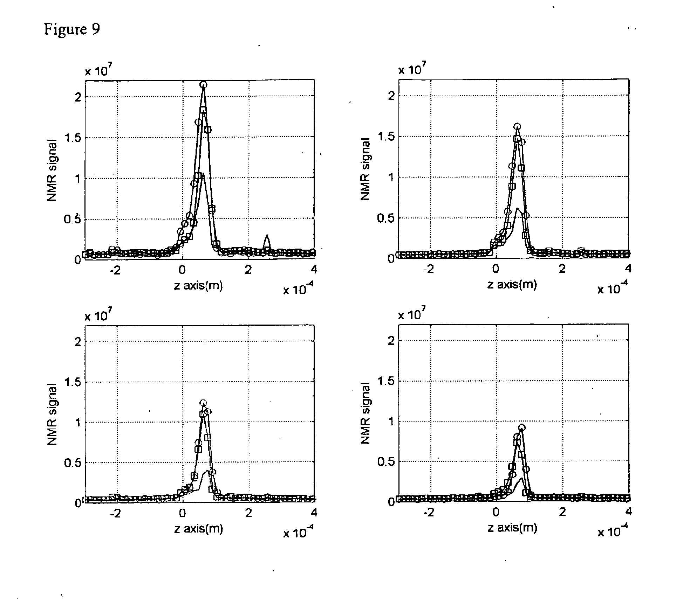

Figure 9 shows four data sets acquired with the four different NMR repetition times with the first panel in the figure corresponding to the shortest time. In this figure, square and circle symbols have been superimposed onto the curves corresponding to the data for the SPIO-labelled spots, whilst the data for the blank spot are indicated by lines without superimposed symbols. The signals from the SPIO-labelled spots are similar to one another and are higher than for the blank spot. The contrast is enhanced for shorter TR, although it remains strong at the longest (400 ms) TR value in these data.

### 4. Data from Experiments using a small bore MRI Instrument Example 4.1 Imaging of a chemical array using water

A glass slide possessing a line of 1 mm SPIO-labelled spots and a line of 0.5 mm SPIO-labelled spots was prepared (see section 2d). This was immersed in water and imaged with increasing spatial resolution.

This data shows that an array of SPIO-labelled, spots may be imaged in a single image acquisition sequence.

### Example 4.2 Imaging of a DNA array using water, ethanol and silicone oil

A glass slide with a 3x4 rectangular array pattern of alternate SPIO-labelled and non-labelled spots of diameter 4 mm was prepared (see sections 2C and 2F). These were imaged using three different fluids. This slide was immersed in i) water, ii) ethanol and iii) silicone oil, as the imaging fluids and slices were taken in two planes, one slightly higher than the other.

In each case, the SPIO-labelled spots were visible while non-labelled spots were not visible. This experiment demonstrates that differences in contrast can be obtained using different imaging fluids with the same array. For a given echo time, TE, the contrast is better for molecules diffusing faster, because the SPIO T₂^{*} relaxation effect is exploited in these data sets.

### Example 4.3 Simultaneous imaging of a stack of slides containing chemical arrays

The experiment in section 4.1 was repeated, but with three slides stacked, with four 160 µm cover slips used as spacers. Spot sizes, as described in section 2d, were-0.5 mm and 1.0 mm spots in a 2x7 array. The top slide had a pattern of alternate SPIO-labelled spot and non-labelled spot on both lines, the middle slide had three SPIO labelled-spots followed by three non-labelled spots on both lines and the bottom slide contained two lines of SPIO labelled-spots Even without optimisation of the slide positions, the spots were detectable and so demonstrate that simultaneous measurement of a plurality of slides (here, three) is possible.

The above experiments demonstrate the use of NMR for detecting interactions on arrays using the examples of both chemical arrays and arrays in which DNA hybridisation takes place. The feasibility of detecting spots on multiple stacked substrates is also disclosed. A number of parameters for carrying out these studies are disclosed and optimised including the choice of slice thickness to optimise contrast, the use of slices located slightly above the substrate surface to help to minimise signal from non-specifically bound SPIO's, and the choice of NMR fluid, with water being better than ethanol and silicone oil. The effect of the wetting properties of the fluid for the substrate is also shown. The studies also show that the effect of SPIOs on the NMR extends vertically around a distance of 1 mm. This is not the extent of the spot, it is the extent of the magnetic field perturbation caused by the SPIOs. This effect also extends horizontally, and its extent can be modulated by changing the sequence's echo time, TE.

## Claims

1. A method of detecting an interaction of one or more target substances present in a fluid on a microarray on a substrate, the method employing
a microarray comprising a plurality of probes immobilised or immobilisable on the substrate at a plurality of locations, wherein the probes are capable of interacting with one or more target substances that may be present in the fluid, wherein the probes and/or the one or more target substances are associated with magnetic nanoparticles,
the method comprising:
(a) contacting the probes with the fluid under conditions in which one or more of the target substances, if present, interact with their respective probes, wherein the fluid comprises spin-carrying molecules detectable by nuclear magnetic resonance (NMR),
(b) where the probes are immobilisable on the substrate, immobilising the probes at locations on the microarray; and
(c) measuring a change in a NMR signal originating from the spin-carrying molecules in the vicinity of the probes at one or more locations in the microarray to determine whether the interaction between a target substance and its respective probes has occurred, wherein the magnetic nanoparticles associated with the probes and/or one or more target substances amplify the change in the NMR signal by introducing a localised magnetic field gradient when the one or more target substances interact with their respective probes, thereby amplifying the NMR relaxation rate of the spin carrying molecules by enhancing their dephasing or causing a loss of coherence of their nuclear spins.

2. The method of claim 1 wherein the magnetic nanoparticles are associated with the one or more target substances.

3. The method of claim 1 or claim 2, wherein the method comprises amplifying the change in the NMR signal by applying an external magnetic field gradient during the measuring step to amplify changes in the NMR relaxation rate caused by the interaction of the probes and target substances affecting the diffusion constant of spin-carrying molecules in the fluid sample.

4. The method of any one of the preceding claims, wherein the magnetic nanoparticle comprises magnetite, maghemite, monocrystalline iron oxide nanoparticles, superparamagnetic iron oxide (SPIO), or a gadolinium (Gd) based compound.

5. The method of any one of the preceding claims, wherein the method comprises amplifying the change in the NMR signal by increasing the surface-enhanced relaxation of the spin-carrying molecules in the fluid caused when target substances interact with their respective probes (T₁ and T₂ changes).

6. The method of claim 5, wherein the surface-enhanced relaxation is caused by the appearance or the disappearance of nanoscale surfaces around the magnetic nanoparticle or probe or target substance on which surface-enhanced relaxation of the spin-carrying molecule in the NMR fluid can occur (T₁ and T₂ changes).

7. A method of detecting an interaction of one or more target substances present in a fluid on a microarray on a substrate, the method employing a microarray comprising a plurality of probes immobilised or immobilisable on the substrate at a plurality of locations,
wherein the probes are capable of interacting with one or more target substances that may be present in the fluid, the method comprising:
(a) contacting the probes with the fluid under conditions in which one or more of the target substances, if present, interact with their respective probes, wherein the fluid comprises spin-carrying molecules detectable by nuclear magnetic resonance (NMR),
(b) where the probes are immobilisable on the substrate, immobilising the probes at locations on the microarray; and
(c) measuring a change in a NMR signal originating from the spin-carrying molecules in the vicinity of the probes at one or more locations in the microarray to determine whether the interaction between a target substance and its respective probes has occurred,
wherein neither the probes nor the one or more target substances are associated with a magnetic component, and
wherein the change in NMR signal is caused or amplified by one or both of
(i) a change the diffusion constant of the spin-carrying molecules due to the interaction of the probes with the one or more target substances; and
(ii) an increase in the surface-enhanced relaxation of the spin-carrying molecules due to the interaction of the probes with the one or more target substances (T₁ and T₂ changes).
wherein when the change in NMR signal is caused or amplified by (i), the method comprises amplifying the change in the NMR signal by applying an external magnetic field gradient during the measuring step to amplify changes in the NMR relaxation rate caused by the interaction of the probes and target substances affecting the diffusion constant of spin-carrying molecules in the fluid sample.

8. The method of claim 7, wherein the target substances are associated with a dendritic molecule or polymer to amplify changes in the diffusion constant of the spin-carrying molecules.

9. The method of any one of the preceding claims which comprises repeating the measuring step using one or more further fluids comprising spin-carrying molecules to obtain a plurality of NMR spectra for the microarray or a part thereof.

10. The method of any one of the preceding claims, wherein the contacting and measuring steps are carried out in the same fluid.

11. The method of any one of claims 1 to 10, wherein the spin-carrying molecules are added to the fluid after the contacting step.

12. The method of any one of the preceding claims, wherein:
(a) the spin-carrying molecules are molecules of the fluid; and/or
(b) the spin-carrying molecule in the fluid is water, an oil, a fluorinated or hyperpolarized gas.

13. The method of any one of the preceding claims, wherein:
(i) the interaction is binding, hybridisation, absorption, cross-linking and/or adsorption of a target substance and a probe; and/or
(ii) the measuring step is carried out while one or more of the target substances interacts with their respective probes; or
(iii) the measuring step is carried out after the target substances have bound to their respective probes.

14. The method of any one of the preceding claims, wherein:
(i) the signal is measured using a NMR-MOUSE^{®} device, a magnetic resonance imaging device, a NMR microscope or a NMR spectrometer; and/or
(ii) the signal is measured using pulsed NMR or continuous wave NMR; and/or
(iii) the changes in the NMR signal are determined by measuring changes in T₁, T₂ and/or T₂^{*}.

15. The method of any one of the preceding claims, wherein:
(i) the probes comprise nucleic acid molecules, proteins, peptides, cells or chemical compounds; and/or
(ii) the nucleic acid is DNA, oligonucleotide, mRNA or cDNA; and/or
(iii) the probes are linked to the substrate by linker groups; and/or
(iv) the microarray is a spotted microarray, a lithographic microarray or a bead microarray; and/or
(v) the microarray is a three dimensional microarray; and/or
(vi) the substrate is formed from glass or a polymer; and/or
(vii) the substrate is porous or comprises a porous layer.

## Patentansprüche

1. Verfahren zur Detektion einer Wechselwirkung einer oder mehrerer Zielsubstanzen, die in einem Fluid vorhanden sind, auf einem Microarray auf einem Substrat, wobei beim Verfahren Folgendes eingesetzt wird:
ein Microarray, das mehrere Sonden umfasst, die an mehreren Stellen auf dem Substrat immobilisiert oder immobilisierbar sind, wobei die Sonden in der Lage sind, mit einer oder mehreren Zielsubstanzen, die gegebenenfalls im Fluid vorhanden sind, wechselzuwirken, wobei die Sonden und/oder die eine oder mehreren Zielsubstanzen mit magnetischen Nanopartikeln assoziiert sind,
wobei das Verfahren Folgendes umfasst:
(a) das Kontaktieren der Sonden mit dem Fluid unter Bedingungen, unter denen eine oder mehrere der Zielsubstanzen, falls vorhanden, mit ihren jeweiligen Sonden wechselwirken, wobei das Fluid einen Spin aufweisende Moleküle umfasst, die durch Kernmagnetresonanz (NMR) detektierbar sind,
(b) wenn die Sonden auf dem Substrat immobilisierbar sind, das Immobilisieren der Sonden an Stellen auf dem Microarray; und
(c) das Messen einer Veränderung eines NMR-Signals, das von den einen Spin aufweisenden Molekülen herrührt, in der Umgebung der Sonden an einer oder mehreren Stellen im Microarray, um zu bestimmen, ob die Wechselwirkung zwischen einer Zielsubstanz und seinen jeweiligen Sonden stattgefunden hat, wobei die mit den Sonden und/oder einer oder mehreren Zielsubstanzen assoziierten magnetischen Nanopartikel die Veränderung des NMR-Signals verstärken, indem sie einen örtlich begrenzten Magnetfeldgradienten einführen, wenn die eine oder mehreren Zielsubstanzen mit ihren jeweiligen Sonden wechselwirken, wodurch die NMR-Relaxationsrate der einen Spin aufweisenden Moleküle erhöht wird, indem ihre Dephasierung gesteigert oder ein Kohärenzverlust ihrer Kernspins ausgelöst wird.

2. Verfahren nach Anspruch 1, wobei die magnetischen Nanopartikel mit der einen oder den mehreren Zielsubstanzen assoziiert sind.

3. Verfahren nach Anspruch 1 oder Anspruch 2, wobei das Verfahren das Verstärken der Veränderung des NMR-Signals durch Anlegen eines externen Magnetfeldgradienten während des Messschritts umfasst, um Veränderungen der NMR-Relaxationsrate zu verstärken, die durch die Wechselwirkung zwischen den Sonden und Zielsubstanzen ausgelöst werden, was sich auf die Diffusionskonstante der einen Spin aufweisenden Moleküle in der Fluidprobe auswirkt.

4. Verfahren nach einem der vorangegangenen Ansprüche, wobei die magnetischen Nanopartikel Magnetit, Maghemit, monokristalline Eisenoxidnanopartikel, superparamagnetisches Eisenoxid (SPIO) oder eine Verbindung auf Gadoliniumbasis (Gd) umfassen.

5. Verfahren nach einem der vorangegangenen Ansprüche, wobei das Verfahren das Verstärken der Veränderung des NMR-Signals durch Steigerung der oberflächenverstärkten Relaxation der einen Spin aufweisenden Moleküle im Fluid umfasst, die auftritt, wenn Zielsubstanzen mit ihren jeweiligen Sonden wechselwirken (T₁- und T₂-Veränderung).

6. Verfahren nach Anspruch 5, wobei die oberflächenverstärkte Relaxation durch das Auftreten oder Verschwinden von Oberflächen mit einer Größe im Nanobereich um das magnetische Nanopartikel oder die Sonde oder die Zielsubstanz verursacht wird, auf denen eine oberflächenverstärkte Relaxation des einen Spin aufweisenden Moleküls im NMR-Fluid stattfinden kann (T₁- und T₂-Veränderung).

7. Verfahren zur Detektion einer Wechselwirkung zwischen einer oder mehreren Zielsubstanzen, die in einem Fluid vorhanden sind, auf einem Microarray auf einem Substrat, wobei beim Verfahren Folgendes eingesetzt wird:
ein Microarray, das mehrere Sonden umfasst, die an mehreren Stellen auf dem Substrat immobilisiert oder immobilisierbar sind, wobei die Sonden in der Lage sind, mit einer oder mehreren Zielsubstanzen, die gegebenenfalls im Fluid vorhanden sind, wechselzuwirken, wobei das Verfahren Folgendes umfasst:
(a) das Kontaktieren der Sonden mit dem Fluid unter Bedingungen, unter denen eine oder mehrere der Zielsubstanzen, falls vorhanden, mit ihren jeweiligen Sonden wechselwirken, wobei das Fluid einen Spin aufweisende Moleküle umfasst, die durch Kernmagnetresonanz (NMR) detektierbar sind,
(b) wenn die Sonden auf dem Substrat immobilisierbar sind, das Immobilisieren der Sonden an Stellen auf dem Microarray; und
(c) das Messen einer Veränderung eines NMR-Signals, das von den einen Spin aufweisenden Molekülen herrührt, in der Umgebung der Sonden an einer oder mehreren Stellen im Microarray, um zu bestimmen, ob die Wechselwirkung zwischen einer Zielsubstanz und seinen jeweiligen Sonden stattgefunden hat,
wobei weder die Sonden noch die eine oder mehreren Zielsubstanzen mit einer magnetischen Komponente assoziiert sind und wobei die Veränderung des NMR-Signals durch eines oder beide der Folgenden verursacht oder verstärkt wird:
(i) eine Veränderung der Diffusionskonstante der einen Spin aufweisenden Moleküle aufgrund der Wechselwirkung zwischen den Sonden und einer oder mehreren Zielsubstanzen; und
(ii) eine Steigerung der oberflächenverstärkten Relaxation der einen Spin aufweisenden Moleküle aufgrund der Wechselwirkung zwischen den Sonden und einer oder mehreren Zielsubstanzen (T₁- und T₂-Veränderung),
wobei, wenn die Veränderung des NMR-Signals durch (i) verursacht oder verstärkt wird, das Verfahren das Verstärken der Veränderung des NMR-Signals durch Anlegen eines externen Magnetfeldgradienten während des Messschritts umfasst, um Veränderungen der NMR-Relaxationsrate zu verstärken, die durch die Wechselwirkung der Sonden und Zielsubstanzen verursacht werden, was sich auf die Diffusionskonstante der einen Spin aufweisenden Moleküle in der Fluidprobe auswirkt.

8. Verfahren nach Anspruch 7, wobei die Zielsubstanzen mit einem dendritischen Molekül oder Polymer assoziiert sind, um Veränderungen der Diffusionskonstante der einen Spin aufweisenden Moleküle zu verstärken.

9. Verfahren nach einem der vorangegangenen Ansprüche, welches das Wiederholen des Messschritts unter Verwendung einer oder mehrerer weiterer Fluide, die einen Spin aufweisende Moleküle enthalten, umfasst, um mehrere NMR-Spektren für das Microarray oder einen Teil davon zu erhalten.

10. Verfahren nach einem der vorangegangenen Ansprüche, wobei der Kontaktierungs- und Messschritt im selben Fluid durchgeführt werden.

11. Verfahren nach einem der Ansprüche 1 bis 10, wobei die einen Spin aufweisenden Moleküle nach dem Kontaktierungsschritt zum Fluid zugesetzt werden.

12. Verfahren nach einem der vorangegangenen Ansprüche, wobei:
(a) die einen Spin aufweisenden Moleküle Moleküle des Fluids sind; und/oder
(b) das einen Spin aufweisende Molekül im Fluid Wasser, ein Öl oder ein fluoriertes oder hyperpolarisiertes Gas ist.

13. Verfahren nach einem der vorangegangenen Ansprüche, wobei:
(i) die Wechselwirkung Bindung, Hybridisierung, Absorption, Vernetzung und/oder Adsorption einer Zielsubstanz und einer Sonde ist; und/oder
(ii) der Messschritt durchgeführt wird, während eine oder mehrere der Zielsubstanzen mit ihren jeweiligen Sonden wechselwirken; und/oder
(iii) der Messschritt durchgeführt wird, nachdem die Zielsubstanzen an ihre jeweiligen Sonden gebunden haben.

14. Verfahren nach einem der vorangegangenen Ansprüche, wobei:
(i) das Signal unter Einsatz einer NMR-MOUSE^{®}-Vorrichtung, einer Magnetresonanzbildgebungsvorrichtung, eines NMR-Mikroskops oder eines NMR-Spektrometers gemessen wird; und/oder
(ii) das Signal unter Einsatz von gepulster NMR oder kontinuierlicher NMR gemessen wird; und/oder
(iii) die Veränderungen des NMR-Signals durch die Messung von Veränderungen von T₁, T₂ und/oder T₂^{*} bestimmt werden.

15. Verfahren nach einem der vorangegangenen Ansprüche, wobei:
(i) die Sonden Nucleinsäuremoleküle, Proteine, Peptide, Zellen oder chemische Verbindungen umfassen; und/oder
(ii) die Nucleinsäure DNA, ein Oligonucleotid, mRNA oder cDNA ist; und/oder
(iii) die Sonden über Linkergruppen an das Substrat gebunden sind; und/oder
(iv) das Microarray ein Spotted-Microarray, ein lithographisches Microarray oder ein Bead-Microarray ist; und/oder
(v) das Microarray ein dreidimensionales Microarray ist; und/oder
(vi) das Substrat aus Glas oder einem Polymer besteht; und/oder
(vii) das Substrat porös ist oder eine poröse Schicht umfasst.

## Revendications

1. Procédé de détection d'une interaction d'une ou plusieurs substances cibles présentes dans un fluide dans un microréseau sur un substrat, le procédé employant
un microréseau comprenant une pluralité de sondes immobilisées ou immobilisables sur le substrat en une pluralité d'emplacements, où les sondes sont capables d'interagir avec une ou plusieurs substances cibles qui peuvent être présentes dans le fluide, où les sondes et/ou les une ou plusieurs substances cibles sont associées à des nanoparticules magnétiques,
le procédé comprenant les étapes consistant à:
(a) mettre en contact les sondes avec le fluide dans des conditions dans lesquelles une ou plusieurs des substances cibles, si elles sont présentes, interagissent avec leurs sondes respectives, où le fluide comprend des molécules portant un spin détectables par résonance magnétique nucléaire (RMN),
(b) là où les sondes sont immobilisables sur le substrat, immobiliser les sondes à des emplacements sur le microréseau; et
(c) mesurer un changement dans un signal RMN ayant pour origine les molécules portant un spin au voisinage des sondes en un ou plusieurs emplacements dans le microréseau pour déterminer si l'interaction entre une substance cible et ses sondes respectives s'est produite, où les nanoparticules magnétiques associées aux sondes et/ou une
ou plusieurs substances cibles amplifient le changement dans le signal RMN en introduisant un gradient de champ magnétique localisé lorsque les une ou plusieurs substances cibles interagissent avec leurs sondes respectives, amplifiant ainsi le taux de relaxation RMN des molécules portant un spin en accentuant leur déphasage ou en entraînant une perte de cohérence de leurs spins nucléaires.

2. Procédé selon la revendication 1, dans lequel les nanoparticules magnétiques sont associées aux une ou plusieurs substances cibles.

3. Procédé selon la revendication 1 ou la revendication 2, dans lequel le procédé comprend l'amplification du changement dans le signal RMN par application d'un gradient de chemin magnétique externe pendant l'étape de mesure pour amplifier des changements dans le taux de relaxation RMN provoqués par l'interaction des sondes et des substances cibles affectant la constante de diffusion des molécules portant un spin dans l'échantillon de fluide.

4. Procédé selon l'une quelconque des revendications précédentes, dans lequel la nanoparticule magnétique comprend la magnétite, la maghémite, des nanoparticules monocristallines d'oxyde de fer, l'oxyde de fer superparamagnétique (SPIO), ou un composé à base de gadolinium (Gd).

5. Procédé selon l'une quelconque des revendications précédentes, dans lequel le procédé comprend l'amplification du changement dans le signal RMN par accroissement de la relaxation accentuée en surface des molécules portant un spin dans le fluide induite lorsque les substances cibles interagissent avec leurs sondes respectives (changements T₁ et T₂).

6. Procédé selon la revendication 5, dans lequel la relaxation accentuée en surface est provoquée par l'apparition ou la disparition de surfaces d'échelle nanométrique autour de la nanoparticule magnétique ou de la sonde ou de la substance cible sur laquelle la relaxation accentuée en surface de la molécule portant un spin dans le fluide RMN peut se produire (changements T₁ et T₂).

7. Procédé de détection d'une interaction d'une ou plusieurs substances cibles présentes dans un fluide sur un microréseau sur un substrat, le procédé employant
un microréseau comprenant une pluralité de sondes immobilisées ou immobilisables sur le substrat en une pluralité d'emplacements, où les sondes sont capables d'interagir avec une ou plusieurs substances cibles qui peuvent être présentes dans le fluide, le procédé comprenant les étapes consistant à:
(a) mettre en contact les sondes avec le fluide dans des conditions dans lesquelles une ou plusieurs des substances cibles, si elles sont présentes, interagissent avec leurs sondes respectives, où le fluide comprend des molécules portant un spin détectables par résonance magnétique nucléaire (RMN),
(b) là où les sondes sont immobilisables sur le substrat, immobiliser les sondes à des emplacements sur le microréseau; et
(c) mesurer un changement dans un signal RMN ayant pour origine les molécules portant un spin au voisinage des sondes en un ou plusieurs emplacements dans le microréseau pour déterminer si l'interaction entre une substance cible et ses sondes respectives s'est produite,
où ni les sondes ni les une ou plusieurs substances cibles ne sont associées à un composant magnétique, et où le changement dans le signal RMN est provoqué ou amplifié par un ou deux éléments parmi
(i) un changement de la constante de diffusion des molécules portant un spin dû à l'interaction des sondes avec les une ou plusieurs substances cibles; et
(ii) une augmentation dans la relaxation accentuée en surface des molécules portant un spin due à l'interaction des sondes avec les une ou plusieurs substances cibles (changements T₁ et T₂) ;
où lorsque le changement dans le signal RMN est provoqué ou amplifié par (i), le procédé comprend l'amplification du changement dans le signal RMN par application d'un gradient de champ magnétique externe pendant l'étape de mesure pour amplifier les changements dans le taux de relaxation RMN provoqués par l'interaction des sondes et des substances cibles affectant la constante de diffusion des molécules portant un spin dans l'échantillon de fluide.

8. Procédé selon la revendication 7, dans lequel les substances cibles sont associées à une molécule ou un polymère dendritique pour amplifier les changements dans la constante de diffusion des molécules portant un spin.

9. Procédé selon l'une quelconque des revendications précédentes, qui comprend la répétition de l'étape de mesure en utilisant un ou plusieurs fluides supplémentaires comprenant des molécules portant un spin pour obtenir une pluralité de spectres RMN pour le microréseau ou une partie de celui-ci.

10. Procédé selon l'une quelconque des revendications précédentes, dans lequel les étapes de contact et de mesure sont réalisées dans le même fluide.

11. Procédé selon l'une quelconque des revendications 1 à 10, dans lequel les molécules portant un spin sont ajoutées au fluide après l'étape de contact.

12. Procédé selon l'une quelconque des revendications précédentes, dans lequel:
(a) les molécules portant un spin sont des molécules du fluide; et/ou
(b) la molécule portant un spin dans le fluide est l'eau, une huile, un gaz fluoré ou hyperpolarisé.

13. Procédé selon l'une quelconque des revendications précédentes, dans lequel:
(i) l'interaction est une liaison, hybridation, absorption, réticulation et/ou adsorption d'une substance cible et d'une sonde; et/ou
(ii) l'étape de mesure est réalisée tandis qu'une ou plusieurs des substances cibles interagissent avec leurs sondes respectives; ou
(iii) l'étape de mesure est réalisée après que les substances cibles se sont liées à leurs sondes respectives.

14. Procédé selon l'une quelconque des revendications précédentes, dans lequel:
(i) le signal est mesuré en utilisant un dispositif NMR-MOUSE^{®}, un dispositif d'imagerie par résonance magnétique, un microscope RMN ou un spectromètre RMN; et/ou
(ii) le signal est mesuré en utilisant une RMN pulsée ou une RMN à onde continue; et/ou
(iii) les changements dans le signal RMN sont déterminés en mesurant les changements dans T₁, T₂ et/ou T₂^{*}.

15. Procédé selon l'une quelconque des revendications précédentes, dans lequel:
(i) les sondes comprennent des molécules d'acide nucléique, des protéines, des peptides, des cellules ou des composés chimiques; et/ou
(ii) l'acide nucléique est l'ADN, un oligonucléotide, l'ARNm ou l'ADNc; et/ou
(iii) les sondes sont liées au substrat par des groupes lieurs; et/ou
(iv) le microréseau est un microréseau maculé, un microréseau lithographique ou un microréseau à billes; et/ou
(v) le microréseau est un microréseau en trois dimensions; et/ou
(vi) le substrat est formé de verre ou d'un polymère; et/ou
(vii) le substrat est poreux ou comprend une couche poreuse.
